# EUROPEAN PATENT APPLICATION

(11) **EP 4 790 723 A1**
(43) Date of publication of application: **12.08.2026**
(21) Application number: 25305174.2
(22) Date of filing: 06.02.2025
(51) Int. Cl.: G16H 50/20, G16H 50/70

(54) **ARTIFICIAL NEURAL NETWORK**

(71) Applicant: Koninklijke Philips N.V., 5656 AE Eindhoven (NL)
(72) Inventor: KHELDOUNI, Amine, 5656 Eindhoven (NL); SHRESHTHA, Kumar, 5656 Eindhoven (NL); CHOFFIN, Benoît, 5656 Eindhoven (NL); PEZZOTTI, Nicola, 5656 Eindhoven (NL); DUPONT, Rémi, 5656 Eindhoven (NL); FACURY DE SOUZA, Luiz, 5656 Eindhoven (NL)
(74) Representative: Philips Intellectual Property & Standards

(57) **Abstract**

Proposed concepts thus aim to provide schemes, solutions, concepts, designs, methods and systems pertaining to training an artificial neural network for interpreting clinical measurement signals. **In** particular, embodiments aim to provide a method for training an artificial neural network for interpreting clinical measurement signals by training the ANN on a plurality of non-manually annotated clinical measurement signals, and then automatically retraining said ANN on additional non-manually annotated clinical measurement signals responsive to a trigger (e.g., a specific time period elapsing and/or a threshold volume of new data in the database being reached).

## Description

### FIELD OF THE INVENTION

This invention relates to the field of training an artificial neural network, and more specifically, to the field of training an artificial neural network for interpreting clinical measurement signals.

### BACKGROUND OF THE INVENTION

Electrocardiograms (ECGs) are a cornerstone in the diagnosis of cardiovascular diseases (CVD). They are valued for their non-invasive nature, low cost, and the information they provide regarding cardiac function. However, despite their widespread use, the interpretation of ECGs remains a complex task that requires significant expertise. Clinicians must understand only the electro-physiology of the heart but also how various cardiac abnormalities manifest in the electrical signals recorded by an ECG. This expertise is not always available in all healthcare settings.

Traditional automated ECG (or other clinical measurement signals, such as EEG, EMG, PPG, etc.) interpretation systems have limitations in terms of performance, often requiring significant manual input and adjustments to adapt to different clinical contexts. Furthermore, some cardiovascular diseases are rare, and even large ECG databases lack positive cases of such abnormalities, which makes training of algorithms to detect such abnormalities even more challenging.

### SUMMARY OF THE INVENTION

The invention is defined by the claims.

According to examples in accordance with an aspect of the invention, there is provided a computer-implemented method for training an artificial neural network for interpreting clinical measurement signals.

The method comprises: training an artificial neural network on a clinical measurement database consisting of a plurality of clinical measurement signals devoid of manual annotations, wherein the training is self-supervised; adding a plurality of supplementary clinical measurement signals devoid of manual annotations to the clinical measurement database to generate an updated clinical measurement database; and retraining the artificial neural network on the updated clinical measurement database responsive to a predetermined trigger.

Proposed concepts thus aim to provide schemes, solutions, concepts, designs, methods and systems pertaining to training an artificial neural network for interpreting clinical measurement signals. In particular, embodiments aim to provide a method for training an artificial neural network (ANN) for interpreting clinical measurement signals by training the ANN on a plurality of non-manually annotated clinical measurement signals, and then automatically retraining said ANN on additional non-manually annotated clinical measurement signals responsive to a trigger (e.g., a specific time period elapsing and/or a threshold volume of new data in the database being reached).

In other words, it is proposed that by not requiring manual annotation of the training data, the ANN can self-learn without supervision on a large quantity of data and so avoid annotation bottlenecks. Furthermore, the ANN can be configured to automatically retrain after/when new unannotated data has been added to a database, thus allowing the ANN to automatically continually improve, and as the new data is unannotated it can be more readily and more frequently added to the database (i.e., due to the lack of annotation bottlenecks) and thus the ANN can be more frequently updated. The (re-)trained ANN can then be used for a variety of purposes such as, for example, being converted/adapted into a targeted diagnostic model.

For example, a Deep Learning pipeline is proposed which leverages large-scale clinical measurement signal databases (such as ECG) to automate the pre-training of a large foundation model. Such a model is capable of continuously improving its clinical measurement signal interpretation capabilities and clinical knowledge (such as cardiology knowledge) without supervision, thus avoiding annotation bottlenecks. The foundation model can then, in turn, be used as a backbone for more focused diagnostic models which are guided by expert manual annotations to detect clinical abnormalities. This training configuration requires fewer manual annotations while still providing an effective model. Alternatively, or additionally, the foundation model can be used itself, for example, to cluster ECG beats or delineate an EEG signal.

The inventors have also realized that by not leveraging manual/human annotations in the training of the foundation model (the ANN), the biases and convention-mismatches introduced by these annotations and/or interpretations can be avoided. Furthermore, the proposed training regime can help to overcome the problem of lack of positive samples for rare but significant diseases and syndromes as well as reducing the laborious and manual intervention required for iterating ANNs.

Given the scarcity of expert interpreters of ECG (or other clinical measurement signals, such as EEG, EMG, PPG, etc.) and the need for timely and precise diagnosis, there is pressing need for automated tools that can assist clinicians interpreting ECGs (or other clinical measurement signals), and this invention aims to help solve this problem.

Ultimately, an improved method for training at least one artificial neural network for interpreting clinical measurement signals is provided.

In some embodiments, the method may further comprise further training the retrained artificial neural network to generate a diagnostic artificial neural network, configured to predict the presence of an indicator of a clinical abnormality in an input clinical measurement signal, based on a manually-annotated clinical measurement database comprising a plurality of manually-annotated clinical measurement signals. In other words, the retrained artificial neural network can be considered a foundation model which can then be fine-tuned / adapted into a diagnostic model using manually-annotated data. This may provide a more effective use of manually-annotated data, resulting in an efficient and effective diagnostic ANN.

In some embodiments, the method may further comprise predicting the presence of an indicator of a clinical abnormality in a first clinical measurement signal of a subject by inputting the first clinical measurement signal into the diagnostic artificial neural network. This may provide an effective and efficient way of predicting whether a clinical measurement signal includes an indicator of a clinical abnormality.

In some embodiments, the diagnostic artificial neural network may be configured to only predict the presence of a single clinical abnormality or one of a single group of clinical abnormalities. Constricting the diagnostic ANN to only predicting a single disease, for example, or one of a single group of diseases, may allow the diagnostic ANN to become more effective for this purpose.

In some embodiments, the number of manually-annotated clinical measurement signals may be fewer than the number of clinical measurement signals devoid of manual annotations. This may allow the generation of effective diagnostic ANNs without requiring a large amount of manually annotated data.

In some embodiments, the method may further comprise: automatically annotating the first clinical measurement signal based on the predicted presence of an indicator of a clinical abnormality in the first clinical measurement signal; and adding the automatically-annotated first clinical measurement signal to the manually-annotated clinical measurement database. This may allow clinical measurement signals machine-annotated by the diagnostic ANN to be added to the training data which can then be used to re-train the diagnostic ANN, which will then go on to machine-annotate further clinical measurement signals, thus resulting in a self-improving loop.

In some embodiments, the method may further comprise using the retrained artificial neural network to analyze a second input clinical measurement signal. This may allow the updated foundation model to be used standalone to analyze clinical measurement signals, for example to cluster ECG beats.

In some embodiments, the retrained artificial neural network analyzing the second input clinical measurement signal may comprise the retrained artificial neural network delineating the second input clinical measurement signal. This may be a particularly useful application of the retrained foundation ANN.

In some embodiments, the method may further comprise extracting features of a first input clinical measurement signal using the retrained artificial neural network. Again, this may be a particularly useful application of the retrained foundation ANN.

In some embodiments, predicting the presence of an indicator of a clinical abnormality may comprise predicting the presence of an indicator of a clinical abnormality in a subject by inputting the extracted features of the first input clinical measurement signal into the diagnostic artificial neural network. This may provide a particularly effective and/or efficient way for it to be predicted whether a clinical measurement signal includes an indicator of a clinical abnormality (e.g., a disease).

In some embodiments, the clinical measurement signals devoid of manual annotations may comprise at least one of: ECG signals; EEG signals; EMG signals; ultrasound signals; PPG signals; and radiography signals. The present invention may be particularly applicable for use with these types of clinical measurement signals.

In some embodiments, the predetermined trigger may comprise at least one of: a predetermined number of supplementary clinical measurement signals devoid of manual annotations being added to the clinical measurement database; and a predetermined period of time passing since the artificial neural network was last trained or retrained. These may allow the ANN to be automatically retrained at suitable intervals.

According to another aspect of the invention, there is provided a computer program comprising code means for implementing the method of any herein disclosed method when said program is run on a processing system.

According to another aspect of the invention, there is provided a system for training an artificial neural network for interpreting clinical measurement signals. The system comprising: a processing arrangement configured to: train an artificial neural network on a clinical measurement database consisting of a plurality of clinical measurement signals devoid of manual annotations, wherein the training is self-supervised; add a plurality of supplementary clinical measurement signals devoid of manual annotations to the clinical measurement database to generate an updated clinical measurement database; and retrain the artificial neural network on the updated clinical measurement database responsive to a predetermined trigger.

In some embodiments, the processing arrangement may be further configured to: further train the retrained artificial neural network to generate a diagnostic artificial neural network, configured to predict the presence of an indicator of a clinical abnormality in an input clinical measurement signal, based on a manually-annotated clinical measurement database comprising a plurality of manually-annotated clinical measurement signals.

Thus, there may be proposed concepts for training an artificial neural network for interpreting clinical measurement signals, and this may be done based on self-supervised training on non-manually annotated clinical measurement signals, and automatically retraining the ANN after new data has been added to the database used for training.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified flow diagram of a method for training an artificial neural network for interpreting clinical measurement signals according to a proposed embodiment;
Fig. 2 is a flow diagram of a method for training an artificial neural network for interpreting clinical measurement signals according to a proposed embodiment;
Fig. 3 is a flow diagram of a method for training an artificial neural network for interpreting clinical measurement signals according to a proposed embodiment; Fig. 4 is a simplified block diagram of a system for training an artificial neural network for interpreting clinical measurement signals according to a proposed embodiment; and
Fig. 5 illustrates an example of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Implementations in accordance with the present disclosure relate to various techniques, methods, schemes and/or solutions pertaining to training an artificial neural network for interpreting clinical measurement signals. According to proposed concepts, a number of possible solutions may be implemented separately or jointly. That is, although these possible solutions may be described below separately, two or more of these possible solutions may be implemented in one combination or another.

Embodiments of the invention aim to provide a method for training an artificial neural network for interpreting clinical measurement signals. This can be achieved by training the ANN on a plurality of non-manually annotated clinical measurement signals, and then automatically retraining said ANN on additional non-manually annotated clinical measurement signals responsive to a trigger (e.g., a specific time period elapsing and/or a threshold volume of new data in the training database being reached).

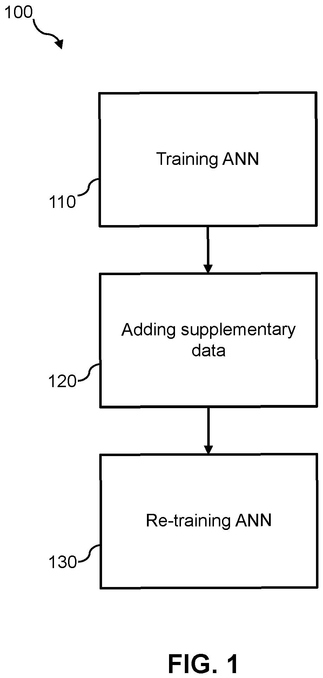

In other words, it is proposed that by not requiring manual annotation, the ANN can self-learn without supervision on a large quantity of data and so avoid annotation bottlenecks. Furthermore, the ANN can be configured to automatically retrain after/when new unannotated data has been added to a database, thus allowing the ANN to automatically continually improve, and as the new data is unannotated it can be more readily and more frequently added to the database (i.e., due to the lack of annotation bottlenecks) and thus the ANN can be more frequently updated. The (re-)trained ANN can then be used for a variety of purposes such as, for example, being converted/adapted into a targeted diagnostic model. Referring now to Fig. 1, there is depicted a simplified flow diagram of a computer-implemented method 100 for training an artificial neural network for interpreting clinical measurement signals according to a proposed embodiment.

The method 100 begins with the step 110 of training an artificial neural network (ANN) on a clinical measurement database consisting of (i.e., exclusively comprising) a plurality of clinical measurement signals devoid of manual annotations (i.e., non-manually annotated clinical measurement signals, i.e., non-human annotated clinical measurement signals). In other words, the clinical measurement database does not include anything other than clinical measurement signals which are not manually annotated - e.g., they can be entirely unannotated or machine-annotated. In some embodiments, the clinical measurement signals are entirely unannotated, i.e., not even machine-annotated. In other words, in some embodiments, step 110 can comprise training an ANN on a clinical measurement database consisting of a plurality of clinical measurement signals devoid of annotations. It should be noted that the clinical measurement database consisting of the plurality of clinical measurement signals devoid of manual annotations does not mean that the number of clinical measurement signals cannot change (as will be seen in step 120) but rather that the database only comprises this type of clinical measurement signal.

As the skilled person would know, an artificial neural network can be understood as a computational model inspired by biological neural networks, consisting of interconnected nodes (neurons) organized in layers that transform input data through weighted connections and activation functions to produce outputs.

As the skilled person would appreciate, a clinical measurement database can take many forms, for instance, local memory, cloud storage, a distributed network of memory units, etc. A clinical measurement signal can be understood as a time-varying quantity that represents a physiological parameter, captured through sensors or monitoring devices, such as heart rate, blood pressure, or electrical activity of organs (ECG, EEG).

For instance, in this embodiment, the clinical measurement signals devoid of manual annotations specifically comprise at least one of: ECG signals; EEG signals; EMG signals; ultrasound signals; PPG signals; and radiography signals. The present invention is particularly applicable for use with these clinical measurement signals, though of course, in other embodiments, any suitable type of clinical measurement signal can be used. Preferably, the clinical measurement signals are restricted to one single type of signal, e.g., ECG signals or EEG signals, however, examples are foreseen where the clinical measurement signals comprise a mixture of types (e.g., both ECG signals and PPG signals), thus resulting in a multi-modal ANN. For instance, a patient's full health history and clinical tests could be input into the retrained ANN, the model then being able to cross-check features and extract information from different medical exams to deliver the most accurate and detailed diagnoses.

The training in step 110 is self-supervised. This means that the model learns from unlabeled data by automatically generating supervisory signals from the data itself, often by predicting masked or held-out portions of the input, enabling learning without manual annotations. The training being self-supervised can also be referred to as unsupervised pre-training; self-training; auto-supervised learning; self-learning; an/or autonomous supervision.

The method 100 also comprises step 120 of adding a plurality of supplementary clinical measurement signals devoid of manual annotations to the clinical measurement database to generate an updated clinical measurement database. In other words, in step 120, two or more further/additional non-manually annotated clinical measurement signals are added to the clinical measurement database such that the updated clinical measurement database includes (as least a substantial majority, if not all) of the clinical measurement signals which were contained in it during step 110 plus the two or more further/additional clinical measurement signals.

The method 100 also comprises step 130 of retraining the artificial neural network on the updated clinical measurement database responsive to a predetermined trigger. In other words, the ANN undergoes additional learning on the new/supplementary/additional clinical measurement signals which have been added to the clinical measurement database. This retraining is triggered by one or more predetermined requirements being met. For example, in this embodiment, the predetermined trigger comprises at least one of: a predetermined number of supplementary clinical measurement signals devoid of manual annotations being added to the clinical measurement database; and a predetermined period of time passing since the artificial neural network was last trained or retrained. These requirements allow the ANN to be automatically retrained at suitable intervals, though as the skilled person would understand, in other embodiments, other suitable requirements/triggers can be used.

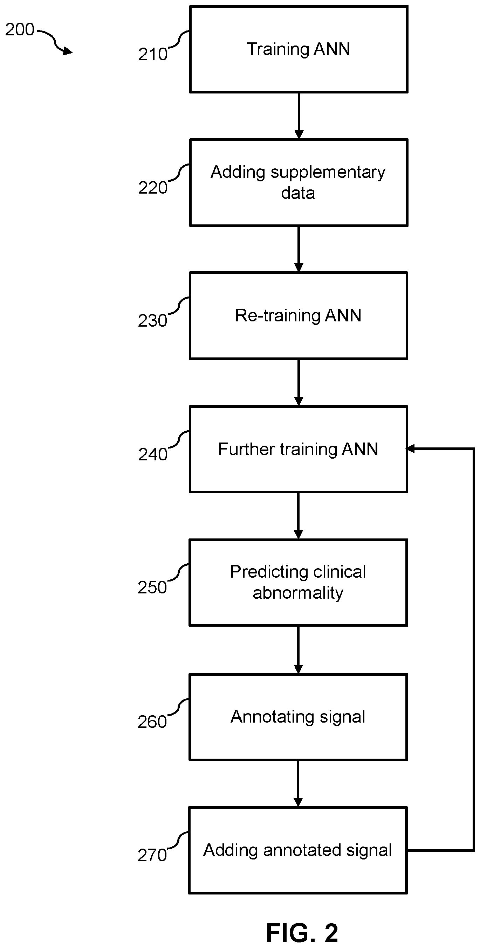

Referring now to Fig. 2, there is depicted flow diagram of a computer-implemented method 200 for training an artificial neural network for interpreting clinical measurement signals according to a proposed embodiment. Steps 210, 220 and 230 are substantially the same as steps 110, 120 and 130 of method 100, respectively.

Step 240 comprises further training the retrained artificial neural network to generate a diagnostic artificial neural network, configured to predict the presence of an indicator of a clinical abnormality in an input clinical measurement signal, based on (i.e., further trained on) a manually-annotated clinical measurement database comprising a plurality of manually-annotated clinical measurement signals. In other words, the retrained artificial neural network can be considered a foundation model, a version/instance of which can then be fine-tuned / adapted into a diagnostic model using manually-annotated data. This provides a more effective use of manually-annotated data, resulting in an efficient and effective diagnostic ANN.

As with the clinical measurement database, the manually-annotated clinical measurement database can take many forms, for instance, local memory, cloud storage, a distributed network of memory units, etc. A manually-annotated clinical measurement signal can be understood as a clinical measurement signal which has been annotated by a human (e.g., an expert clinician).

A clinical abnormality can be understood as a measurable or observable deviation from expected physiological parameters, reference ranges, or established patterns that may indicate a pathological condition, disease or disorder.

In this embodiment, the diagnostic artificial neural network is configured to only predict the presence of a single clinical abnormality or one of a single group of clinical abnormalities. Constricting the diagnostic ANN to only predicting a single disease, for example, or one of a single group of diseases, allows the diagnostic ANN to become more effective for this purpose. A single clinical abnormality can also be understood as a single disease, a single pathology, a single condition or a single disorder. A single group of clinical abnormalities can be understood as a collection of distinct medical conditions that share common characteristics, such as affected organ systems, underlying pathophysiological mechanisms, symptoms, or causative factors. As the skilled person would appreciate, however, this feature is not essential and in other embodiments, the diagnostic ANN can be configured to predict the presence of a plurality of (unrelated) clinical abnormalities.

In other embodiments, step 240 can comprise further training a plurality of instances of the retrained artificial neural network to generate a plurality of diagnostic artificial neural networks, each configured to predict the presence of an indicator of a different single clinical abnormality or a different single group of clinical abnormalities, based on at least one manually-annotated clinical measurement database comprising a plurality of manually-annotated clinical measurement singles. **In** some embodiments, the further training of the multiple instances of the retrained ANN may be based on multiple clinical measurement databases, each comprising a plurality of manually-annotated clinical measurement signals corresponding to a different single clinical abnormality or a different single group of clinical abnormalities.

In this embodiment, the number of manually-annotated clinical measurement signals is fewer than the number of clinical measurement signals devoid of manual annotations. This allows the generation of effective diagnostic ANNs without requiring a large amount of manually annotated data. For instance, in this embodiment, the number of manually-annotated clinical measurement signals is, in fact, less than 1/100^{th} the number of clinical measurement signals devoid of manual annotations.

In other words, the foundation model can be fine-tuned into multiple diagnostic model versions. This constitutes new/further training on top of the foundation model, but does not require many samples (e.g., a few-shot learning setup) where the objective is to guide the foundation model towards a specific task (e.g., diagnosing a specific clinical abnormality). Therefore, a few additional parameters are essentially built on top of the foundation model layers that are needed to train towards the specific task. A new diagnostic model can be generated for each new task but using the same foundation model.

Step 250 comprises predicting the presence of an indicator of a clinical abnormality in a first clinical measurement signal of a subject by inputting the first clinical measurement signal into the diagnostic artificial neural network. This provides an effective and efficient way of predicting whether a clinical measurement signal includes an indicator of a clinical abnormality, and therefore, in essence, predicting whether the subject has the clinical abnormality.

Step 260 comprises automatically annotating the first clinical measurement signal based on the predicted presence of an indicator of a clinical abnormality in the first clinical measurement signal. In other words, step 260 comprises machine-annotating the first clinical measurement signal based on the presence of an indicator of a clinical abnormality in the first clinical measurement signal predicted in step 250. For instance, in this embodiment, the diagnostic ANN which predicted the presence of the indicator of the clinical abnormality in the first clinical measurement signal automatically/machine annotates the first clinical measurement signal accordingly. In other embodiments, however, another machine-learning model or system can annotate the first clinical measurement signal based on the ANN's prediction, i.e., after having been fed (i.e., had input) the ANN's prediction and the first clinical measurement signal.

Step 270 comprises adding the automatically-annotated first clinical measurement signal to the manually-annotated clinical measurement database (and the method can thus iteratively loop from step 240 onwards). This allows clinical measurement signals machine-annotated by the diagnostic ANN (or using the diagnostic ANN's predictions) to be added to the training data which can then be used to re-train the diagnostic ANN, which will then go on to machine-annotate further clinical measurement signals, thus resulting in a self-improving loop.

In some embodiments, step 270 can further comprise adding the automatically-annotated first clinical measurement signal to the updated clinical measurement database (and the method can thus iteratively loop from 230 onwards). This would allow clinical measurement signals machine-annotated by the diagnostic ANN (or using the diagnostic ANN's predictions) to be added to the training data which would then be used to re-train the foundation ANN, and which can then be used to generate updated diagnostic ANNs, thus resulting in a self-improving loop.

Essentially, in this invention, an approach is proposed which leverages a large pre-trained deep learning model for clinical measurement signal analysis, and particularly, for ECG analysis. This invention thus aims at reducing the need for expertly (human) annotated data. For example, the pre-trained model with self-supervision associates an ECG with an embedding learned on millions of cases in a self-supervised fashion. No human annotation is required, but the proposed example way of training the model is key to obtain meaningful embeddings in a cardiology sense (i.e., able to correlate with cardiac conditions). The key to the proposed method is that a database of unannotated data (which is periodically updated) can be leveraged to keep an updated version of a pre-trained (foundation) model. For example, at set times (e.g., every week), the large foundation model can be trained to include new ECGs uploaded to the database. Hence, the foundation model continuously keeps on learning in a self-supervised manner from the incoming ECGs in the database. The model can then be converted into diagnostic models using a small but high-quality manually-annotated ECG dataset, e.g., to frame the output space of the algorithm's diagnostics. These annotations can be, for example, ECG diagnostics interpreted by expert cardiologists, which are very valuable but tedious to acquire, especially in large volumes. The diagnostic models can be used, however, to machine-annotate clinical measurement signals which can then be put back into their training database to allow the diagnostic models to continually retrain as the training database grows. This design thus essentially constitutes a feedback loop for the diagnostic model. In some embodiments, the machine-annotated clinical measurement signals can also be added to the updated clinical measurement database to allow the foundation model to retrain based on this provided data too. This helps the core foundation algorithm strengthen its interpretation capabilities and in time, include new diseases that were very rare (if present at all) in the original database.

In a specific example of the invention, an ever-increasing number of diagnostic models can be trained starting from a pre-trained large foundation model. The training of the foundation model is embedded into an operating cycle through an automated retraining pipeline, as herein described, by new data uploaded to the database. The resulting foundation model can thus generate latent embeddings which capture features of input clinical measurement signals - these embeddings offer a compressed representation of the clinical measurement signal and its intrinsic characteristics.

A variety of downstream tasks can be derived from the (re)trained foundation model. For instance, multiple diagnostic models can be produced which can then be used to supplement the database used for training via interpretations and analyses of data (e.g., via machine-annotations). The foundation model itself can also be used directly, for example, to detect and/or classify cardiac abnormalities, to delineate ECG signals, to detect waves, to generate interpretability maps and patches for clinical measurement signals, and/or for ECG clustering for cross patients.

For instance, in a specific example of the invention, the foundation model can be used to generate a scatter plot where each data point represents an ECG in the latent space. Using dimensionality reduction and clustering, some meaningful clusters can be derived, such as a cluster of ECGs which all have similar pathologies. For example, all ECGs in said cluster represent ECGs of patients with a complete left bundle branch block (CLBBB) which represents a significant cardiac abnormality. Hence, the complete left bundle branch block pattern is well noticed by the foundation model and grouped as a specific cluster that reflects this QRS complex widening and morphology changes, typical of ventricular conduction delay patterns without any supervision.

Leveraging learned embeddings, the foundation model can, for example, generate diagnostic-focused classifiers that target specific clinical abnormalities such as cardiac arrhythmias, myocardial infarction, or other electrophysiological disorders. By analyzing the latent embeddings, the model can identify patterns of anomalies which are indicative of specific conditions. This approach has been found not only to improve the accuracy of detection in a standard setup, but has also been found to outperform standard supervised training using only manual annotations.

For instance, a scarcity analysis was conducted where the positive samples of a given cardiac abnormality (Brugada syndrome) were reduced in a dataset. In this study, a few-shot learning setup was used where few Brugada syndrome datapoints were stored in a training database and the effects of the few-shot learning on a system according to the invention (e.g., a pre-trained foundation model which was then finetuned on a focused Brugada target) and a standard system (e.g., using fully supervised learning) were studied.

The area under the curve (AUC) was higher overall when using the proposed inventive system on a small amount of data. The gap in performance between the two approaches was even more notable when only 10-25% of the positive samples (i.e., 6 to 17 true cases of patients affected by Brugada syndrome) were used. Also, the proposed inventive system managed to reach the same AUC of 96% with half the number of positive samples as the fully supervised model which required 100% of the positive samples to reach such performance. Based on this analysis, the proposed inventive system is seen to outperform the standard setup where no self-supervised foundation model is used. Hence, the self-supervised foundation model enriches the diagnostic model with more robustness regarding the annotated data, faster convergence, and improved performance for clinical abnormalities detection.

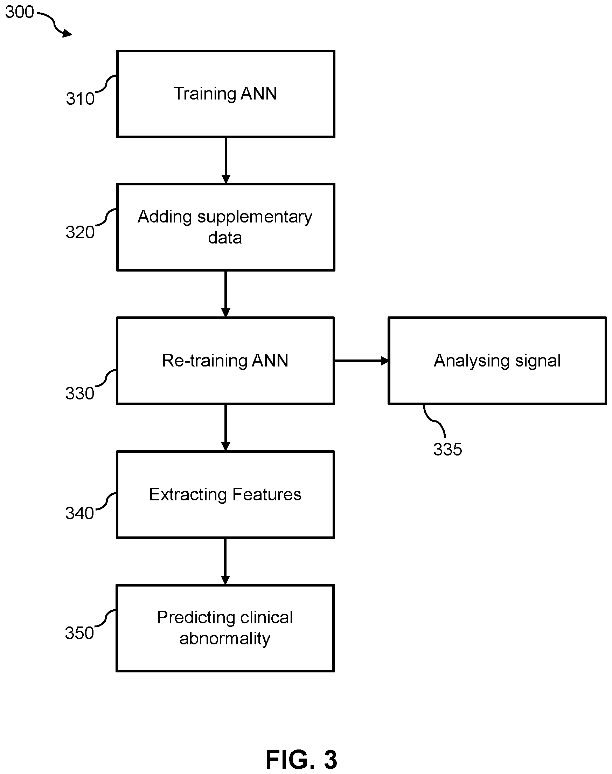

Referring now to Fig. 3, there is depicted a flow diagram of a method 300 for training an artificial neural network for interpreting clinical measurement signals according to a proposed embodiment. Steps 310, 320 and 330 are substantially the same as steps 110, 120 and 130 of method 100, respectively.

Step 335 comprises using the retrained artificial neural network to analyze a second input clinical measurement signal. This allows the updated foundation model to be used standalone to analyze clinical measurement signals, for example, to cluster ECG beats. As the skilled person would understand, the terms 'first' and 'second' when referring to the input clinical measurement signals are merely arbitrary labels for differentiation and are not limiting in any way. For instance, the first and second clinical measurement signals may be the same clinical measurement signal or may be different clinical measurement signals.

In some embodiments, the retrained artificial neural network analyzing the second input clinical measurement signal (in step 335) may comprise the retrained artificial neural network delineating the second input clinical measurement signal. This may be a particularly useful application of the retrained foundation ANN, especially then the input clinical measurement signal comprises an ECG signal.

Step 340 comprises extracting features of a first input clinical measurement signal using the retrained artificial neural network. Again, this is a particularly useful application of the retrained foundation ANN. Extracting features of a first input clinical measurement signal can be understood as the process of identifying and determining relevant characteristics or patterns from raw physiological data to create a simplified representation that captures key diagnostic or prognostic information while reducing dimensionality.

Step 350 comprises predicting the presence of an indicator of a clinical abnormality in a subject by inputting the extracted features of the first input clinical measurement signal into the diagnostic artificial neural network. This provides a particularly effective and/or efficient way for it to be predicted whether a clinical measurement signal includes an indicator of a clinical abnormality (e.g., a disease).

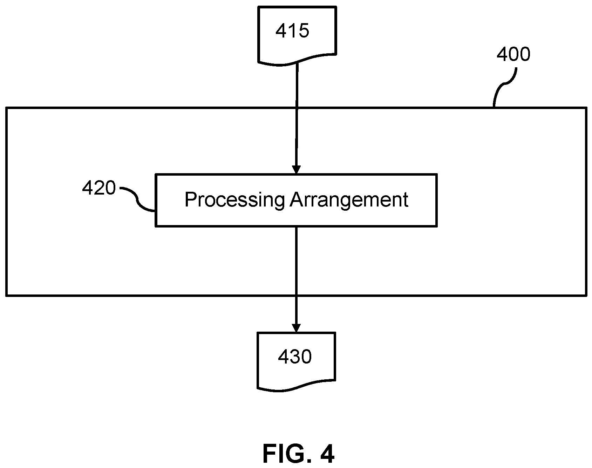

Referring now to Fig. 4, there is depicted a system 400 for training an artificial neural network for interpreting clinical measurement signals according to a proposed embodiment. The system 400 comprises a processing arrangement 420.

The processing arrangement 420 is configured to perform any herein-disclosed method, such as method 100, 200, or 300. In this embodiment, the method is configured to: train an artificial neural network on a clinical measurement database consisting of a plurality of clinical measurement signals devoid of manual annotations (the plurality of clinical measurement signals being the input 415), wherein the training is self-supervised; add a plurality of supplementary clinical measurement signals devoid of manual annotations to the clinical measurement database to generate an updated clinical measurement database; and retrain the artificial neural network on the updated clinical measurement database responsive to a predetermined trigger (wherein the retrained artificial neural network can be considered the output 430).

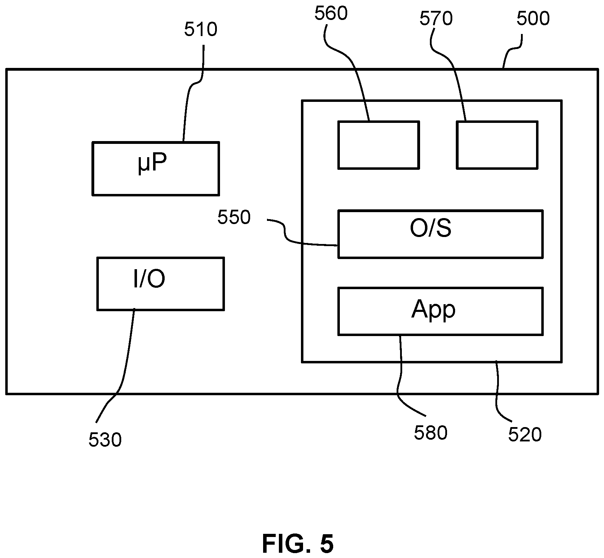

Fig. 5 illustrates an example of a computer 500 within which one or more parts of an embodiment may be employed. Various operations discussed above may utilize the capabilities of the computer 500. In this regard, it is to be understood that system functional blocks can run on a single computer or may be distributed over several computers and locations (e.g. connected via internet).

The computer 500 includes, but is not limited to, PCs, workstations, laptops, PDAs, palm devices, servers, storages, and the like. Generally, in terms of hardware architecture, the computer 500 may include one or more processors 510, memory 520 and one or more I/O devices 530 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 510 is a hardware device for executing software that can be stored in the memory 520. The processor 510 can be virtually any custom made or commercially available processor, a central processing unit (CPU), a digital signal processor (DSP), or an auxiliary processor among several processors associated with the computer 500, and the processor 510 may be a semiconductor based microprocessor (in the form of a microchip) or a microprocessor.

The memory 520 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 520 may incorporate electronic, magnetic, optical, and/or other types of storage media. Note that the memory 520 can have a distributed architecture, where various components are situated remote from one another, but can be accessed by the processor 510.

The software in the memory 520 may include one or more separate programs, each of which comprises an ordered listing of executable instructions for implementing logical functions. The software in the memory 520 includes a suitable operating system (O/S) 550, compiler 560, source code 570, and one or more applications 580 in accordance with exemplary embodiments. As illustrated, the application 580 comprises numerous functional components for implementing the features and operations of the exemplary embodiments. The application 580 of the computer 500 may represent various applications, computational units, logic, functional units, processes, operations, virtual entities, and/or modules in accordance with exemplary embodiments, but the application 580 is not meant to be a limitation.

The operating system 550 controls the execution of other computer programs, and provides scheduling, input-output control, file and data management, memory management, and communication control and related services. It is contemplated by the inventors that the application 580 for implementing exemplary embodiments may be applicable on all commercially available operating systems.

Application 580 may be a source program, executable program (object code), script, or any other entity comprising a set of instructions to be performed. When a source program, then the program is usually translated via a compiler (such as the compiler 560), assembler, interpreter, or the like, which may or may not be included within the memory 520, so as to operate properly in connection with the O/S 550. Furthermore, the application 580 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 530 may include input devices such as, for example but not limited to, a mouse, keyboard, scanner, microphone, camera, etc. Furthermore, the I/O devices 530 may also include output devices, for example but not limited to a printer, display, etc. Finally, the I/O devices 530 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 530 also include components for communicating over various networks, such as the Internet or intranet.

If the computer 500 is a PC, workstation, intelligent device or the like, the software in the memory 520 may further include a basic input output system (BIOS) (omitted for simplicity). The BIOS is a set of essential software routines that initialize and test hardware at start-up, start the O/S 550, and support the transfer of data among the hardware devices. The BIOS is stored in some type of read-only-memory, such as ROM, PROM, EPROM, EEPROM or the like, so that the BIOS can be executed when the computer 500 is activated.

When the computer 500 is in operation, the processor 510 is configured to execute software stored within the memory 520, to communicate data to and from the memory 520, and to generally control operations of the computer 500 pursuant to the software. The application 580 and the O/S 550 are read, in whole or in part, by the processor 510, perhaps buffered within the processor 510, and then executed.

When the application 580 is implemented in software it should be noted that the application 580 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 580 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figs. 1-3, and the system of Fig. 4, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Fig. 5 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention.

## Claims

1. A computer-implemented method (100) for training an artificial neural network for interpreting clinical measurement signals, the computer-implemented method comprising:
training an artificial neural network (110) on a clinical measurement database consisting of a plurality of clinical measurement signals devoid of manual annotations, wherein the training is self-supervised;
adding a plurality of supplementary clinical measurement signals devoid of manual annotations to the clinical measurement database (120) to generate an updated clinical measurement database; and
retraining the artificial neural network (130) on the updated clinical measurement database responsive to a predetermined trigger.

2. The computer-implemented method of claim 1, further comprising: further training the retrained artificial neural network (240) to generate a diagnostic artificial neural network, configured to predict the presence of an indicator of a clinical abnormality in an input clinical measurement signal, based on a manually-annotated clinical measurement database comprising a plurality of manually-annotated clinical measurement signals.

3. The computer-implemented method of claim 2, further comprising: predicting the presence of an indicator of a clinical abnormality in a first clinical measurement signal of a subject (250) by inputting the first clinical measurement signal into the diagnostic artificial neural network.

4. The computer-implemented method of claim 2 or 3, wherein the diagnostic artificial neural network is configured to only predict the presence of a single clinical abnormality or one of a single group of clinical abnormalities.

5. The computer-implemented method of any of claims 2 to 4, wherein the number of manually-annotated clinical measurement signals is fewer than the number of clinical measurement signals devoid of manual annotations.

6. The computer-implemented method of any of claims 3 to 5, further comprising:
automatically annotating the first clinical measurement signal (260) based on the predicted presence of an indicator of a clinical abnormality in the first clinical measurement signal; and
adding the automatically-annotated first clinical measurement signal (270) to the manually-annotated clinical measurement database.

7. The computer-implemented method of any prior claim, further comprising: using the retrained artificial neural network to analyze a second input clinical measurement signal (335).

8. The computer-implemented method of claim 7, wherein the retrained artificial neural network analyzing the second input clinical measurement signal comprises the retrained artificial neural network delineating the second input clinical measurement signal.

9. The computer-implemented method of any prior claim, further comprising extracting features of a first input clinical measurement signal (340) using the retrained artificial neural network.

10. The computer-implemented method of claim 9, dependent on any of claims 3 to 6, wherein predicting the presence of an indicator of a clinical abnormality comprises:
predicting the presence of an indicator of a clinical abnormality in a subject (350) by inputting the extracted features of the first input clinical measurement signal into the diagnostic artificial neural network.

11. The computer-implemented method of any prior claim, wherein the clinical measurement signals devoid of manual annotations comprise at least one of: ECG signals; EEG signals; EMG signals; ultrasound signals; PPG signals; and radiography signals.

12. The computer-implemented method of any prior claim, wherein the predetermined trigger comprises at least one of: a predetermined number of supplementary clinical measurement signals devoid of manual annotations being added to the clinical measurement database; and a predetermined period of time passing since the artificial neural network was last trained or retrained.

13. A computer program product comprising computer program code means which, when executed on a computing device having a processing system, cause the processing system to perform all of the steps of the computer-implemented method according to any of claims 1 to 12.

14. A system (400) for training an artificial neural network for interpreting clinical measurement signals, the system comprising:
a processing arrangement (420) configured to:
train an artificial neural network on a clinical measurement database consisting of a plurality of clinical measurement signals devoid of manual annotations, wherein the training is self-supervised;
add a plurality of supplementary clinical measurement signals devoid of manual annotations to the clinical measurement database to generate an updated clinical measurement database; and
retrain the artificial neural network on the updated clinical measurement database responsive to a predetermined trigger.

15. The system of claim 14, wherein the processing arrangement is further configured to:
further train the retrained artificial neural network to generate a diagnostic artificial neural network, configured to predict the presence of an indicator of a clinical abnormality in an input clinical measurement signal, based on a manually-annotated clinical measurement database comprising a plurality of manually-annotated clinical measurement signals.
